# EUROPÄISCHE PATENTSCHRIFT

(11) **EP 1 814 572 B1**
(45) Veröffentlichungstag und Bekanntmachung des Hinweises auf die Patenterteilung: **02.06.2010**
(21) Anmeldenummer: 05800013.4
(22) Anmeldetag: 03.11.2005
(51) Int. Cl.: A61K 38/15, A61P 33/00

(54) **DEPSIPEPTIDE ZUR VERHINDERUNG VERTIKALER ENDOPARASITEN-INFEKTIONEN**
DEPSIPEPTIDES FOR PREVENTION OF VERTICAL ENDOPARASITE INFECTIONS
DEPSIPEPTIDES POUR LA PREVENTION D'INFECTIONS VERTICALES PROVOQUEES PAR DES ENDOPARASITES

(30) Priorität: 16.11.2004 DE 102004055316
(43) Veröffentlichungstag der Anmeldung: 08.08.2007
(73) Patentinhaber: Bayer Animal Health GmbH, 51368 Leverkusen (DE)
(72) Erfinder: BACH, Thomas, 42349 Wuppertal (DE); TÄNZLER, Janina, 55268 Nieder-Olm (DE)
(86) Internationale Anmeldenummer: PCT/EP2005/011748
(87) Internationale Veröffentlichungsnummer: WO 2006/053641

(56) Entgegenhaltungen:
- EP-A- 0 626 375
- EP-A- 1 142 577
- DE-A1- 4 317 458
- BIALEK RALF ET AL: "Parasitic disease in pregnancy and congenital parasitosis: Part II: Helminthic infections" ZEITSCHRIFT FUER GEBURTSHILFE UND NEONATOLOGIE, Bd. 203, Nr. 3, Mai 1999 (1999-05), Seiten 128-133, XP009062020 ISSN: 0948-2393

## Beschreibung

Die vorliegende Erfindung betrifft die Verwendung endoparasitizider Depsipeptide zur Herstellung von Arzneimitteln zur Verhinderung der vertikalen Infektion mit Endoparasiten.

Das cyclische Depsipeptid PF1022 und seine Wirkung gegen Endoparasiten ist bekannt aus EP-A 382 173. Weitere cyclische Depsipeptide und ihre endoparasitizide Wirkung sind Gegenstand der Patentanmeldungen EP-A 626 376; EP-A 626 375; EP-A 644 883.

Endoparasitizide Mittel enthaltend Praziquantel oder Epsiprantel und cyclische Depsipeptide sind in EP 662 326 beschrieben.

Es ist bekannt, dass diese Mittel nach oraler, parenteraler oder transdermaler Applikation gegen Endoparasiten des betreffenden Tieres wirken. Bislang war aber nicht bekannt, dass diese Wirkstoffe auch vertikale Infektionen bei den Nachkommen der Tiere verhindern können.

Die Erfindung betrifft daher:

Die Verwendung von endoparasitiziden Depsipeptiden zur Herstellung von Arnzneimitteln zur Verhinderung der vertikalen Infektion mit Endoparasiten.

Depsipeptide sind den Peptiden ähnlich und unterscheiden sich von diesen darin, daß ein oder mehrere α-Aminosäurebausteine durch α-Hydroxycarbonsäurebausteine ersetzt sind. Erfindungsgemäß bevorzugt eingesetzt werden cyclische Depsipeptide mit 18 bis 24 Ringatomen, insbesondere mit 24 Ringatomen.

Zu den Depsipeptiden mit 18 Ringatomen zählen Verbindungen der allgemeinen Formel (I): 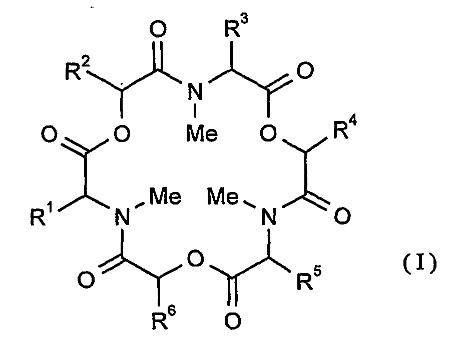 in welcher
- R¹, R³ und R⁵: unabhängig voneinander für Wasserstoff geradkettiges oder verzweigtes Alkyl mit bis zu 8 Kohlenstoffatomen, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie gegebenenfalls substituiertes Arylalkyl, wobei als Substituenten genannt seien Halogen, Hydroxy, Alkyl und Alkoxy, stehen,
- R², R⁴ und R⁶: unabhängig voneinander für Wasserstoff, geradkettiges oder verzweigtes Alkyl mit bis zu 8 Kohlenstoffatomen, Hydroxyalkyl, Mercaptoalkyl, Alkanoyloxyalkyl, Alkoxyalkyl, Aryloxyalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Alkoxycarbonylaminoalkyl, Alkenyl, Cycloalkyl, Cycloalkylalkyl gegebenenfalls substituiertes Aryl oder Arylalkyl wobei als Substituenten genannt seien Halogen, Hydroxy, Alkyl, Alkoxy, stehen, sowie deren optische Isomere und Racemate.

Bevorzugt sind Verbindungen der Formel (I), 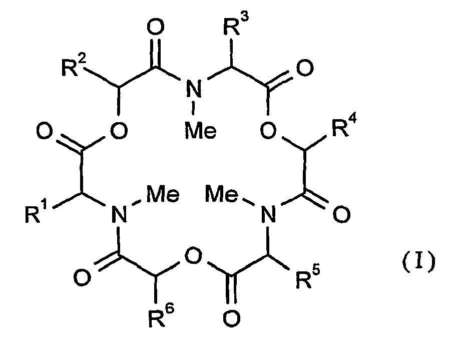 in welcher
- R¹, R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxy-ethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, Carboary-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, Guanido-C₁-C₆-alkyl, insbesondere Guanidopropyl, C₁-C₄-Alkoxycarbonylamino-C₁-C₆-alkyl, insbesondere tert.-Butoxycarbonylaminopropyl, tert.-Butoxycarbonylaminobutyl, 9-Fluorenylmethoxycarbonyl-(Fmoc)amino-C₁-C₆-alkyl, insbesondere 9-Fluorenyl-methoxycarbonyl(Fmoc)aminopropyl, 9-Fluorenylmethoxycarbonyl(Fmoc)aminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl-C₁-C₄-alkyl, insbesondere Phenylmethyl das gegebenenfalls durch Reste aus der Reihe Halogen, insbesondere Fluor, Chlor Brom oder Iod, Hydroxy, C₁-C₄- Alkoxy, insbesondere Methoxy oder Ethoxy, C₁-C₄-Alkyl, insbesondere Methyl, substituiert sein kann,
- R², R⁴ und R⁶: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxy-ethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl, Phenyl-C₁-C₄-alkyl, insbesondere Phenylmethyl das gegebenenfalls durch Reste aus der Reihe Halogen, insbesondere Fluor, Chlor Brom oder Iod, Hydroxy, C₁-C₄- Alkoxy, insbesondere Methoxy oder Ethoxy, C₁-C₄-Alkyl, insbesondere Methyl, substituiert sein kann, sowie deren optische Isomere und Racemate.

Besonders bevorzugt sind Verbindungen der Formel (I),
in welcher
- R¹, R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxyethyl, C₁-C₄-Alkoxycarbonylamino-C₁-C₆-alkyl, insbesondere tert.-Butoxycarbonylaminopropyl, tert.-Butoxycarbonylaminobutyl, C₂-C₈-Alkenyl, insbesondere Vinyl, Allyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl-C₁-C₄-alkyl, insbesondere Phenylmethyl das gegebenenfalls durch einen oder mehrere gleiche oder verschiedene der oben angegebenen Reste substituiert sein kann,
- R², R⁴ und R⁶: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₆-akyl, insbesondere Hydroxymethyl, Aryl-C₁-C₄-alkyloxy-C₁-C₆-alkyl, insbesondere Benzyloxymethyl, 1-Benzyloxyethyl, Carboxy-C₁-C₆-alkyl, insbesondere Carboxymethyl, Carboxyethyl, C₁-C₄-Alkoxycarbonyl-C₁-C₆-alk-yl, insbesondere Methoxycarbonylmethyl, Ethoxycarbonylethyl, C₁-C₄-Aryl-alkoxycarbonyl-C₁-C₆-alkyl, insbesondere Benzyloxycarbonylmethyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl, Phenyl-C₁-C₄-alkyl, insbesondere Phenylmethyl das gegebenenfalls durch einen oder mehrere gleiche oder verschiedene der oben angegebenen Reste substituiert sein kann,
sowie deren optische Isomere und Racemate.

Ganz besonders bevorzugt sind Verbindungen der Formel (I),
in welcher
- R¹, R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl C₂-C₈-Alkenyl, insbesondere Allyl, C₃-C₇-Cycloalkyl-C₁-C₄-alkyl, insbesondere Cyclohexylmethyl, Phenyl-C₁-C₄-alkyl, insbesondere Phenylmethyl,
- R², R⁴ und R⁶: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, C₂-C₈- Alkenyl, insbesondere Vinyl, Allyl, C₃-C₇-Cycloakyl-C₁-C₄-alkyl, insbesondere Cyclohexylmethyl, Phenyl-C₁-C₄-akyl, insbesondere Phenylmethyl das gegebenenfalls durch einen oder mehrere gleiche oder verschiedene der oben angegebenen Reste substituiert sein kann,
sowie deren optische Isomere und Racemate.

Im einzelnen seien folgende Verbindungen der allgemeinen Formel (I) genannt, in welcher die Reste R¹ bis R⁶ die folgende Bedeutung haben:

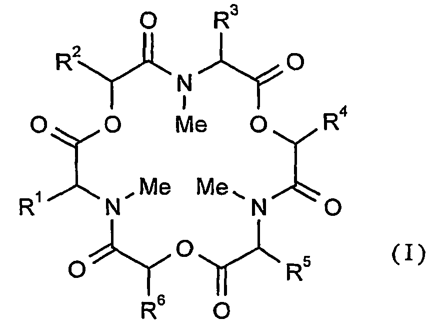

| **R¹** | **R¹** | **R³** | **R⁴** | **R⁵** | **R⁶** |
|---|---|---|---|---|---|
| -CHMeCH₂Me | -Cyclohexyl | -CHMeCH₂Me | -Me | -CHMeCH₂Me | -Me |
| -CHMeCH₂Me | -Cyclohexyl | -CHMeCH₂Me | -Me | -CHMeCH₂Me | -Cyclohexyl |
| -CHMeCH₂Me | -CH₂-Phe | -CHMeCH₂Me | -Me | -CHMeCH₂Me | -Me |
| -CHMeCH₂Me | -CH₂-Phe | -CHMeCH₂Me | -Me | -CHMeCH₂Me | -CH₂-Phe |
| -CHMeCH₂Me | -(CH₂)₃-Me | -CHMeCH₂Me | -Me | -CHMeCH₂Me | -Me |
| -CHMeCH₂Me | -(CH₂)₃-Me | -CHMeCH₂Me | -Me | -CHMeCH₂Me | -(CH₂)₃-Me |
| -CHMe₂ | -CH₂-Phe | -CHMeCH₂Me | -Me | -CHMeCH₂Me | -Me |
| -CH₂-Phe | -CHMe₂ | -CH₂-Phe | -CHMe₂ | -CHMeCH₂Me | -CHMe₂ |
| -CH₂CHMe₂ | -CH₂-Phe | -CH₂CHMe₂ | -Me | -CH₂CHMe₂ | -CH₂-Phe |
| -(CH₂)₃-Me | -Me | -CHMeCH₂Me | -Me | -CHMeCH₂Me | -Me |
| -CHMe₂ | -Me | -CHMe₂ | -Me | -CHMe₂ | -Me |
| -CH₂-Me | -Me | -CH₂-Me | -Me | -CH₂-Me | -Me |
| -(CH₂)₂-Me | -Me | -(CH₂)₂-Me | -Me | -(CH₂)₂-Me | -Me |
| -(CH₂)₃-Me | -Me | -(CH₂)₃-Me | -Me | -(CH₂)₃-Me | -Me |
| -CH₂-CH=CH₂ | -Me | -CH₂-CH=CH₂ | -Me | -(CH₂)-CH=CH₂ | -Me |
| -CHMeCH₂Me | -Me | -CHMeCH₂Me | -Me | -CHMeCH₂Me | -CH₂-Me |
| -CHMeCH₂Me | -Me | -CHMeCH₂Me | -Me | -CHMeCH₂Me | -(CH₂)₂-Me |
| -CHMeCH₂Me | -Me | -CHMeCH₂Me | -Me | -CHMeCH₂Me | -(CH₂)₃-Me |
| -CHMeCH₂Me | -Me | -CM4eCH₂Me | -Me | -CH₂Me | -Me |
| -CHMeCH₂Me | -Me | -CHMeCH₂Me | -Me | -(CH₂)₂-Me | -Me |
| -Cyclohexyl | -Me | -Cyclohexyl | -Me | -Cyclohexyl | -Me |
| -CH₂CHMe₂ | -Cyclohexyl | -CH₂CHMe₂ | -Me | -CH₂CHMe₂ | -Cyclohexyl |
| -CH₂CHMe₂ | -Cyclohexyl | -CH₂CHMe₂ | -Me | -CH₂CEMe₂ | -Me |
| -CHMeCH₂Me | -CHMe₂ | -CHMeCH₂Me | -CHMe₂ | -CHMeCH₂Me | -Me |
| -CH₂-Phe | -Me | -CH₂-Phe | -Me | -CH₂-Phe | -Me |
| -Cyclohexyl | -Me | -Cyclohexyl | -Me | -Cyclohexyl | -Me |
| -CHMe₂ | -CHMe₂ | -CHMe | -Me | -CHMe₂ | -Me |
| -CHMe₂ | -CHMe₂ | -CHMe₂ | -CHMe₂ | -CHMe₂ | -Me |
| -CH₂-Me | -CHMe₂ | -CH₂Me | -Me | -CH₂-Me | -Me |
| -CH₂-Me | -CHMe₂ | -CHMe₂ | -CHMe₂ | -CH₂-Me | -Me |
| -(CH₂)₂-Me | -CHMe₂ | -(CH₂)₂-Me | -Me | -(CH₂)₂-Me | -Me |
| -(CH₂)₂-Me | -CHMe₂ | -(CH₂)₂-Me | -CHMe₂ | -(CH₂)₂-Me | -Me |
| -(CH₂)₃-Me | -CHMe₂ | -(CH₂)₃-Me | -Me | -(CH₂)₃-Me | -Me |
| -(CH₂)₃-Me | -CHMe₂ | -(CH₂)₃-Me | -CHMe₂ | -(CH₂)₃-Me | -Me |
| -CH₂-CH=CH₂ | -CHMe₂ | -CH₂-CH=CH₂ | -Me | -CH₂-CH=CH₂ | -Me |
| -CH₂-CH=CH₂ | -CHMe₂ | -CH₂-CH=CH₂ | -CHMe₂ | -CH₂-CH=CH₂ | -Me |
| -Me | -Me | -CHMeCH₂Me | -Me | -CH₂-Me | -Me |
| -Me | -Me | -CHMeCH₂Me | -Me | -(CH₂)₃-Me | -Me |

| | | | | | |
|---|---|---|---|---|---|
| Me = Methyl; Phe = Phenyl | | | | | |

Weiterhin sei als Depsipeptid die aus EP-OS 382 173 bekannte Verbindung PF 1022 der folgenden Formel (IIa) genannt:

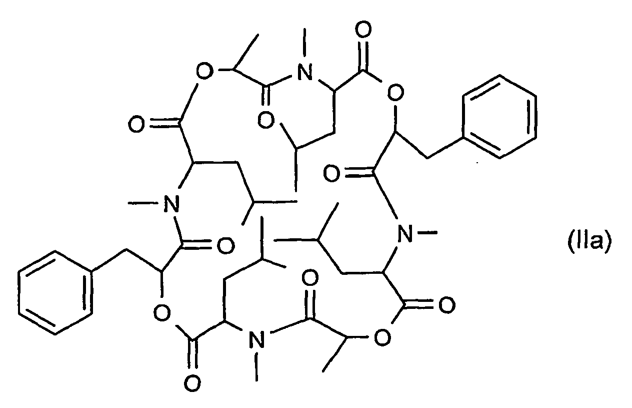

Außerdem seien als Depsipeptide die aus der PCT-Anmeldung WO 93/19053 bekannten Verbindungen genannt.

Insbesondere seien aus WO 93/19053 die Verbindungen der folgenden Formel (IIb) genannt: 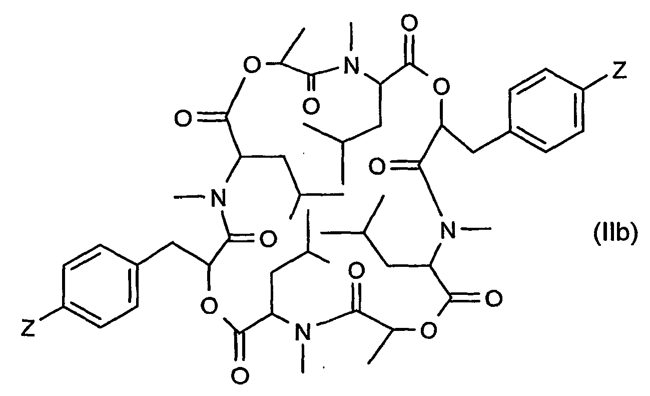 in welcher
- Z: für N-Morpholinyl, Amino, Mono- oder Dimethylamino steht.

Außerdem seien Verbindungen der folgenden Formel (IIc) genannt: 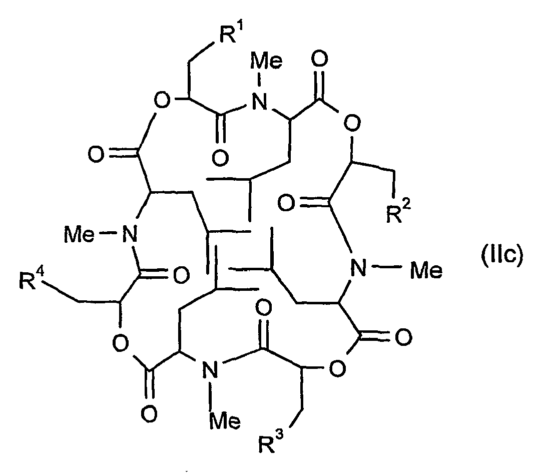 in welcher
- R¹, R², R³, R⁴: unabhängig voneinander für Wasserstoff, C₁-C₁₀-Alkyl oder Aryl, insbesondere Phenyl stehen, die gegebenenfalls substituiert sind durch Hydroxy, C₁-C₁₀-Alkoxy oder Halogen.

Die Verbindungen der allgemeinen Formel (I) sind bekannt und können nach den in EP-A-382 173, DE-A 4 317 432, DE-A 4 317 457, DE-A 4 317 458, EP-A-634 408, EP-A-718 293, EP-A-872 481, EP-A-685 469, EP-A-626 375, EP-A-664 297, EP-A-669 343, EP-A-787 141, EP-A-865 498, EP-A-903 347 beschriebenen Verfahren erhalten werden.

Zu den cyclischen Depsipeptiden mit 24 Ringatomen zählen auch Verbindungen der allgemeinen Formel (IId) 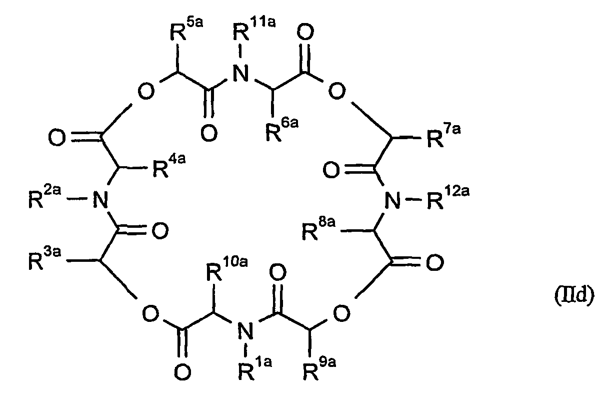 in welcher
- R^{1a}, R^{2a}, R^{11a} und R^{12a}: unabhängig voneinander für C₁₋₈-Alkyl, C₁₋₈-Halogenalkyl, C₃₋₆-Cycloalkyl, Aralkyl, Aryl stehen,
- R^{3a}, R^{5a}, R^{7a}, R^{9a}: unabhängig voneinander für Wasserstoff oder geradkettiges oder verzweigtes C₁₋₈-Alkyl steht, das gegebenenfalls durch Hydroxy, C₁₋₄-Alkoxy, Carboxy, 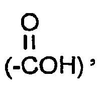 Carboxamid, 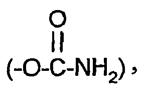 Imidazolyl, Indolyl, Guanidino, -SH oder C₁₋₄-Alkylthio substituiert sein kann und ferner für Aryl oder Aralkyl die durch Halogen, Hydroxy, C₁₋₄-Alkyl, C₁₋₄-Alkoxy substituiert sein können, steht,
- R^{4a}, R^{6a}, R^{8a}, R^{10a}: unabhängig voneinander für Wasserstoff, geradkettiges C₁₋₅-Alkyl, C₂₋₆-Alkenyl, C₃₋₇-Cycloalkyl, die gegebenenfalls durch Hydroxy, C₁₋₄-Alkoxy, Carboxy, Carboxamid, Imidazolyl, Indolyl, Guanidino, SH oder C₁₋₄-Alkylthio substituiert sein können, sowie für Aryl oder Aralkyl die durch Halogen, Hydroxy, C₁₋₄-Alkyl, C₁₋₄-Alkoxy substituiert sein können, stehen
sowie deren optische Isomere und Racemate.

Bevorzugt werden Verbindungen der Formel (IId) eingesetzt, in welcher
- R^{1a}, R^{2a}, R^{11a} und R^{12a}: unabhängig voneinander für Methyl, Ethyl, Propyl, Isopropyl, n-, s-, t-Butyl oder Phenyl, das gegebenenfalls substituiert ist durch Halogen, C₁₋₄-Alkyl, OH, C₁₋₄-Alkoxy, sowie für Benzyl oder Phenylethyl stehen, die gegebenenfalls durch die bei Phenyl angegebenen Reste substituiert sein können;
- R^{3a} bis R^{10a}: die oben angegebene Bedeutung haben.

Besonders bevorzugt sind Verbindungen der Formel (IId), in welcher
- R^{1a}, R^{2a}, R^{11a} und R^{12a}: unabhängig voneinander für Methyl, Ethyl, Propyl, Isopropyl oder n-, s-, t-Butyl stehen,
- R^{3a}, R^{5a}, R^{7a}, R^{9a}: für Wasserstoff, geradkettiges oder verzweigtes C₁₋₈-Alkyl, insbesondere Methyl, Ethyl, Propyl, i-Propyl, n-, s-, t-Butyl, die gegebenenfalls durch C₁₋₄-Alkoxy, insbesondere Methoxy, Ethoxy, Imidazolyl, Indolyl oder C₁₋₄-Alkylthio, insbesondere Methylthio, Ethylthio substituiert sein können, ferner für Phenyl, Benzyl oder Phenethyl stehen, die gegebenenfalls durch Halogen insbesondere Chlor substituiert sein können.
- R^{4a}, R^{6a}, R^{8a}, R^{10a}: unabhängig voneinander für Wasserstoff, Methyl, Ethyl, n-Propyl, n-Butyl, Vinyl, Cyclohexyl, die gegebenenfalls durch Methoxy, Ethoxy, Imidazolyl, Indolyl, Methylthio, Ethylthio substituiert sein können, sowie für Isopropyl, s-Butyl ferner für gegebenenfalls halogensubstituiertes Phenyl, Benzyl oder Phenylethyl stehen.

Die Verbindungen der Formel (IId) können ebenfalls nach den in EP-A-382 173, DE-A 4 317 432, DE-A 4 317 457, DE-A 4 317 458, EP-A-634 408, EP-A-718 293, EP-A-872 481, EP-A-685 469, EP-A-626 375, EP-A-664 297, EP-A-669 343, EP-A-787 141, EP-A-865 498, EP-A-903 347 beschriebenen Verfahren erhalten werden.

Erfindungsgemäß ganz besonders bevorzugte Depsipeptide sind PF 1022 A (siehe Formel (IIa) und Emodepside (PF 1022-221, Verbindung der Formel (II6) worin beide Reste Z für den Morpholinylrest stehen). Der INN Emodepside steht für die Verbindung mit dem systematischen Namen: Cyclo[(*R*)-lactoyl-*N*-methyl-L-leucyl-(*R*)-3-(*p*-morpholinophenyl)lactoyl-*N*-methyl-L-leucyl-(*R*)-lactoyl-*N*-methyl-L-leucyl-(*R*)-3-(*p*-morpholinophenyl)lactoyl-*N*-methyl-L-leucyl.

Wirkstoffe können je nach Struktur in stereoisomeren Formen oder als Stereoisomerengemische vorliegen, z.B. als Enantiomere oder Racemate. Sowohl die Stereoisomerengemische als auch die reinen Stereoisomeren können erfindungsgemäß verwendet werden.

Weiterhin können gegebenenfalls verwendet werden: Salze der Wirkstoffe mit pharmazeutisch annehmbaren Säuren oder Basen und auch Solvate, insbesondere Hydrate, der Wirkstoffe oder ihrer Salze.

Erfindungsgemäß können pathogene Endoparasiten bekämpft werden, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern usw.) vermindert werden, so dass durch den Einsatz der Wirkstoffe eine wirtschaftlichere und einfachere Tierhaltung möglich ist. Zu den pathogenen Endoparasiten zählen generell Cestoden, Trematoden, Nematoden, Acantocephalen. Vorzugsweise lassen sich mit Depsipeptiden Infektionen mit Nematoden bekämpfen; im Einzelnen seien folgende Endoparasiten genannt:
Aus der Ordnung der Enoplida z.B.: Trichuris spp., Capillaria spp., Trichomosoides spp., Trichinella spp..
Aus der Ordnung der Rhabditia z.B.: Micronema spp., Strongyloides spp..
Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp.,
Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp.
Aus der Ordnung der Oxyurida z.B.: Oxyuris spp., Enterobius spp., Passalurus spp., Syphacia spp., Aspiculuris spp., Heterakis spp.
Aus der Ordnung der Ascaridia z.B.: Ascaris spp., Toxascaris spp., Toxocara spp., Parascaris spp., Anisakis spp., Ascaridia spp., Baylisascaris spp..
Aus der Ordnung der Spirurida z.B.: Gnathostoma spp., Physaloptera spp., Thelazia spp., Gongylonema spp., Habronema spp., Parabronema spp., Draschia spp., Dracunculus spp.
Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp.
Aus der Ordnung der Gigantorhynchida z.B.: Filicollis spp., Moniliformis spp., Macracanthorhynchus spp., Prosthenorchis spp.

Erfindungsgemäß bevorzugt ist der Einsatz gegen Infektionen mit Helminthen aus der Ordnung der Rhabditia, z.B. Strongyloides spp.; aus der Ordnung der Strongylida, z.B. Ancylostoma spp., sowie gegen Infektionen gegen die oben bei der Ordnung der Ascaridia genannten Helminthen. Besonders bevorzugt ist der Einsatz gegen Toxocara spp., z.B. Toxocara canis.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen. Ganz besonders bevorzugt ist die Anwendung am Hund.

Bei der erfindungsgemäßen Verwendung erwiesen sich die Depsipeptide als gut verträglich, und zwar sowohl bezüglich der Muttertiere als auch bezüglich der Nachkommen.

Vertikale Infektion bedeutet, dass die Übertragung vom Muttertier auf die Nachkommen stattfindet, und zwar insbesondere pränatal, d.h. bereits im Mutterleib, und/oder galaktogen, also über die Milch. Von besonderer Bedeutung ist die Verhinderung der pränatalen Infektion.

"Verhinderung der pränatalen oder galaktogenen Infektion" steht nicht nur für eine vollständige Verhinderung der Infektion sondern auch für eine unvollständige Verhinderung, bei der das Infektionsrisiko lediglich verringert wird und/oder die Infektion abgeschwächt ist. Anzustreben ist eine mindestens 90 %ige Wirksamkeit.

Die Anwendung der Wirkstoffe erfolgt direkt oder in Form von geeigneten Zubereitungen vorzugsweise enteral, parenteral oder dermal (transdermal) beim Muttertier.

Die enterale Anwendung der Wirkstoffe geschieht z.B. oral in Form von Pulver, Zäpfchen, Tabletten, Kapseln, Fasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspensionen zur oralen oder dermalen Anwendung sowie zur Injektion; halbfeste Zubereitungen;
Formulierungen bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramusculär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen, Baden oder Waschen) aufgebracht. Diese Lösungen werden wie oben bei den Injektionslösungen beschrieben hergestellt.

Es kann vorteilhaft sein, bei der Herstellung Verdickungsmittel zuzufügen.

Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, dass eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglykole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglycolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfat, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektion angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase gelöst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäure der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Cypryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a..

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglykol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien, Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben Angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker-, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Bevorzugte Applikationsformen sind oral, z.B. durch eine geeignete Tablette, oder transdermal, z.B. durch eine geeignete Spot-on Formulierung.

Die erfindungsgemäßen Mittel können zusätzlich Synergisten oder weitere Wirkstoffe, z.B. solche die gegen pathogene Endoparasiten wirken, enthalten. Solche Wirkstoffe sind z.B. L-2,3,5,6-Tetrahydro-6-phenyl-imidazothiazol, Benzimidazolcarbamate wie Febantel, ferner Pyrantel, Epsiprantel oder makrocyclische Lactone wie z.B. Avermectin, Ivermectin oder Selamectin. Besonders bevorzugt ist Praziquantel als Kombinationspartner, insbesondere zusammen mit Emodepsid.

Praziquantel ist als Wirkstoff gegen Endoparasiten lange bekannt (vgl. z. B US 4 001 411). Die vorteilhafte Kombination von Depsipeptiden mit Praziquantel oder Epsiprantel ist in EP-A-662 326 beschrieben.

Kombinationen von Depsipeptiden mit Piperazinen sind in EP-A-1 189 615 beschrieben.

Transdermal applizierbare Mittel enthaltend cyclische Depsipeptide zur Bekämpfung von Endoparasiten sind in unserer ebenfalls anhängigen deutschen Patentanmeldung Akz. 10358525.7 beschrieben.

Anwendungsfertige Zubereitungen enthalten die Wirkstoffe im Allgemeinen in Konzentrationen von jeweils 0,01 - 25 Gew.-%, bevorzugt von 0,1-20 Gewichtsprozent.

Die cyclischen Depsipeptide werden üblicherweise in Mengen von 0,1 bis 8 Gew.-%, bevorzugt 1 bis 6 Gew.-% eingesetzt.

Praziquantel wird üblicherweise in Mengen von 1 bis 25 Gew.-%, bevorzugt 5 bis 15 Gew.-%, besonders bevorzugt 6 bis 14 Gew.-% eingesetzt.

Die Mittel werden durch Mischen der Komponenten in den entsprechenden Mengen in geeigneten Geräten hergestellt. Vorzugsweise geht man dabei so vor, dass man die flüssigen Komponenten mischt und dann die festen Komponenten zugibt und eine homogene Lösung herstellt.

Im allgemeinen hat es sich als vorteilhaft erwiesen Mengen der erfindungsgemäßen Mischung von etwa 0,1 bis etwa 20 mg Wirkstoff je kg Körpergewicht pro Tag zur Erzielung wirksamer Ergebnisse zu verabreichen. Bevorzugt sind 0,5 bis 10 mg Wirkstoff je kg Körpergewicht.

Die folgenden Beispiele erläutern die Erfindung ohne sie einzuschränken.

### Biologisches Beispiel

Die Untersuchungen erfolgten an achtzehn experimentell mit *Toxocara canis* infizierten Beaglehündinnen. Von diesen dienten sechs Tiere als unbehandelte Kontrollgruppe. Zwei unterschiedliche Behandlungsschemata wurden angewendet. In der Behandlungsgruppe 1 (B1) wurden sechs Hündinnen ab dem 42. Trächtigkeitstag täglich bis zur Geburt und in der Behandlungsgruppe 2 (B2) sechs Hündinnen ab dem 42. Trächtigkeitstag alle drei Tage bis zur Geburt der Welpen mit jeweils 1 mg Emodepsid / kg Körpergewicht oral als Tablettenformulierung behandelt. Die Beurteilung der Wirksamkeit der Behandlung erfolgte durch die Ermittlung von drei Zielgrößen bei den Welpen: Anzahl somatischer Larven von *T. canis* in Verdauungsproben der Leber, Lunge, Niere, Magen-Darmtrakt inklusive Inhalt und Muskulatur (Euthanasie der Welpen direkt nach der Geburt), Anzahl ausgeschiedener Eier pro Gramm Kot und Anzahl intestinaler Stadien von *T. canis* nach Darmauswaschung (Euthanasie der Welpen am 35. Lebenstag).

Die prozentuale Wirksamkeit von Emodepsid in B1 betrug für die Zielgröße Anzahl ausgeschiedener Eier pro Gramm Kot 99,9 %, für die Zielgröße Anzahl somatischer Larven in den Verdauungsorganen 96,4 % und für die Zielgröße Anzahl intestinaler Stadien im Darm 99,9 %. In B2 wurde für die Zielgröße Anzahl ausgeschiedener Eier pro Gramm Kot ebenfalls eine Wirksamkeit von 99,9 % erreicht. Für die Zielgröße Anzahl somatischer Larven wurde mit dem Behandlungsschema 2 eine prozentuale Wirksamkeit von Emodepsid von 93,8 % und für die Zielgröße Anzahl intestinaler Stadien im Darm von 98,7 % erzielt. In beiden Behandlungsschemata lag die prozentuale Wirksamkeit von Emodepsid für die einzelnen Zielgrößen sowie auch der Mittelwert aus diesen Zielgrößen in jeder Behandlungsgruppe (B1= 98,7 %, B2= 97,5 %) deutlich über der von der International Cooperation on Harmonisation of Technical Requirements for Registration of Veterinary Medicinal Products (VICH) Guideline 7 geforderten prozentualen Mindestwirksamkeit von 90 % für anthelminthisch wirksame Substanze. Mit beiden Behandlungsschemata wurde eine deutliche Reduktion der pränatalen *T. canis* Infektion bei den Welpen erreicht.

Es wurden weder bei dem Muttertier noch bei den Welpen Unverträglichkeitserscheinungen beobachtet.

## Patentansprüche

1. Verwendung, von endoparasitiziden Depsipeptiden zur Herstellung von Arzneimitteln zur Verhinderung der vertikalen Infektion mit Endoparasiten.

2. Verwendung gemäß Anspruch 1, wobei ein cyclisches Depsipeptid mit 24 Ringatomen verwendet wird.

3. Verwendung gemäß Anspruch 2, wobei als Depsipeptid die Verbindung PF 1022 der Formel (IIa): 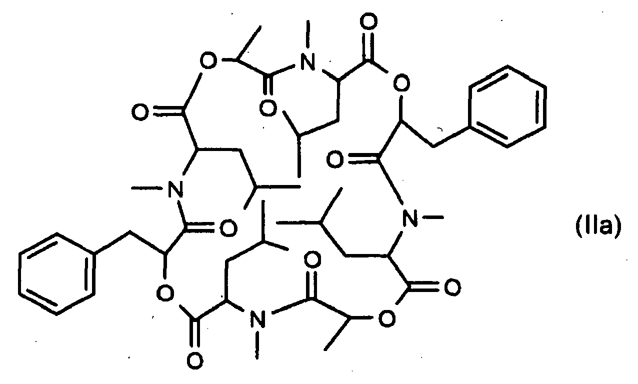 oder eine Verbindung der Formel (IIb): 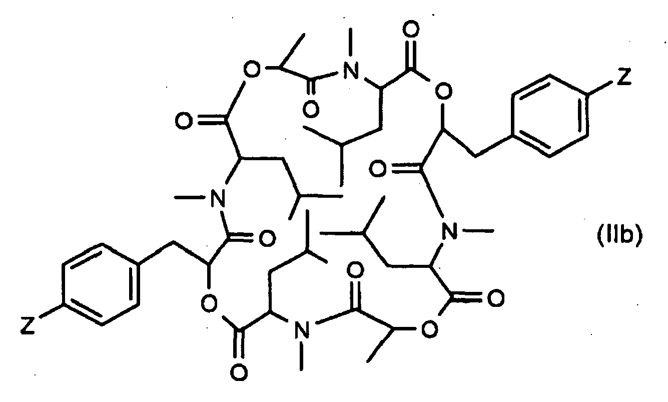 in welcher
Z für N-Morpholinyl, Amino, Mono- oder Dimethylamino steht,
verwendet wird

4. Verwendung gemäß Anspruch 3, wobei als endoparasitizides Depsipeptid Emodepside eingesetzt wird.

5. Verwendung gemäß Anspruch 3, wobei als endoparasitizides Depsipeptid PF 1022 eingesetzt wird.

6. Verwendung gemäß einem der vorstehenden Ansprüche, zur Verhinderung der vertikalen Infektion mit Endoparasiten bei Hunden.

## Claims

1. Use of endoparasiticidal depsipeptides for producing pharmaceuticals for preventing vertical infection with endoparasites.

2. Use according to Claim 1, wherein a cyclic depsipeptide having 24 ring atoms is used.

3. Use according to Claim 2, wherein the depsipeptide used is the compound PF 1022 of the formula (IIa): 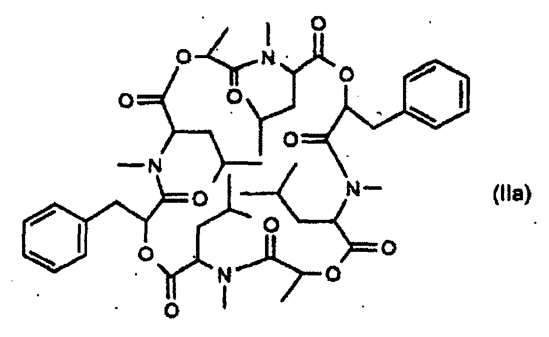 or a compound of the formula (IIb); 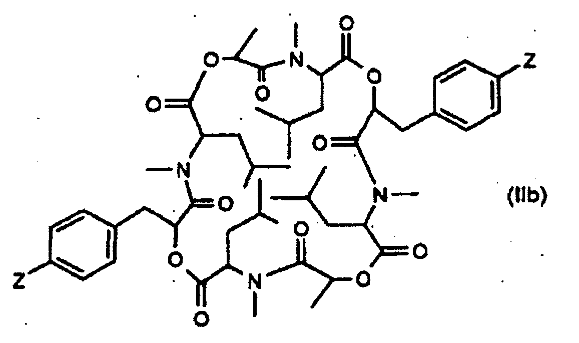 in which
Z is N-morpholinyl, amino, mono- or dimethylamino.

4. Use according to Claim 3, wherein the endoparasiticidal depsipeptide employed is emodepside.

5. Use according to Claim 3, wherein the endoparasiticidal depsipeptide employed is PF 1022.

6. Use according to one of the preceding claims, for preventing vertical infection with endoparasites in dogs.

## Revendications

1. Utilisation de depsipeptides endoparasiticides pour la préparation de médicaments destinés à la prévention de l'infection verticale par des endoparasites.

2. Utilisation selon la revendication 1, où l'on utilise un depsipeptide cyclique ayant 24 atomes cycliques.

3. Utilisation selon la revendication 2, où l'on utilise comme depsipeptide le composé PF 1022 de formule (IIa) : 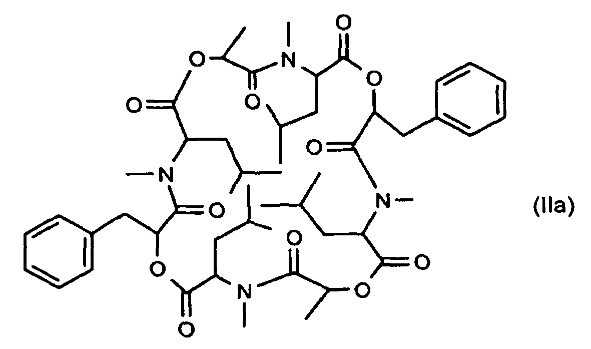 ou un composé de formule (IIb) : 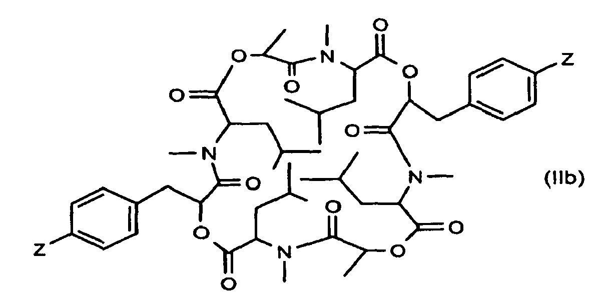 où Z représente N-morpholinyle, amino, mono- ou diméthylamino.

4. Utilisation selon la revendication 3, où on emploie émodepside comme depsipeptide endoparasiticide.

5. Utilisation selon la revendication 3, où on emploie PF 1022 comme depsipeptide endoparasiticide.

6. Utilisation selon l'une des revendications précédentes, destinée à prévenir l'infection verticale par des endoparasites chez le chien.
